# EUROPÄISCHE PATENTSCHRIFT

(11) **EP 2 577 123 B1**
(45) Veröffentlichungstag und Bekanntmachung des Hinweises auf die Patenterteilung: **09.04.2014**
(21) Anmeldenummer: 11706215.8
(22) Anmeldetag: 24.02.2011
(51) Int. Cl.: F16K 15/14, A61M 39/24

(54) **RÜCKSCHLAGVENTIL, INSBESONDERE FÜR MEDIZINISCHE ANWENDUNGEN**
CHECK VALVE, IN PARTICULAR FOR MEDICAL APPLICATIONS
VANNE DE CONTRÔLE, DESTINÉE EN PARTICULIER À DES APPLICATIONS MÉDICALES

(30) Priorität: 01.06.2010 DE 102010022410
(43) Veröffentlichungstag der Anmeldung: 10.04.2013
(73) Patentinhaber: Illinois Tool Works, Inc., Glenview, Cook County IL 60025 (US)
(72) Erfinder: COLM, Michael Carmody, Listowel, Co. Kerry (IE); CASEY, Brendan, Thurles, Co. Tipperary (IE); HENN, Gerard Gabriel, Limerick, Co. Limerick (IE)
(74) Vertreter: Beck, Alexander
(86) Internationale Anmeldenummer: PCT/EP2011/052763
(87) Internationale Veröffentlichungsnummer: WO 2011/151090

(56) Entgegenhaltungen:
- EP-A2- 1 099 457
- WO-A1-96/24791
- DE-U1-202006 016 730
- DE-U1-202008 001 077

## Beschreibung

### Technisches Gebiet

Die Erfindung betrifft ein Rückschlagventil, insbesondere für medizinische Anwendungen, mit einem ersten Schlauchanschlussgehäuse und einem zweiten Schlauchanschlussgehäuse und einer zwischen den beiden Schlauchanschlussgehäusen angeordneten Membranscheibe aus flexiblem Werkstoff, welche beim Überdruck in einem Eingangsdurchlass im ersten Schlauchanschlussgehäuse von einem ringförmigen, einen mit dem Eingangsdurchlass verbundenen Einlassraum umgebenden Ventilsitz abhebbar ist und die bei Überdruck in einem Ausgangsdurchlass sicher und in Minimalzeiten auf dem Ventilsitz andrückbar ist, wobei die Membranscheibe an ihrem äußersten Umfangsbereich mit einem Ringwulst versehen ist, welcher in einander gegenüberliegenden Ringnuten der Schlauchanschlussgehäuse aufgenommen ist, wobei die Membranscheibe radial außerhalb des Ventilsitzes mit Öffnungen versehen ist, die mit einem Auslassraum verbunden sind.

### Stand der Technik

Ein Rückschlagventil dieser Bauart ist nach einem früheren Vorschlag der Anmelderin aus dem deutschen Gebrauchsmuster 20 2006 016 730.7 bekannt. Dieses bekannte Ventil ist insbesondere für höhere Drücke ausgelegt.

Ein weiteres Rückschlagventil mit den oben genannten Merkmalen ist ferner noch ebenfalls nach einem früheren Vorschlag der Anmelderin aus dem deutschen Gebrauchsmuster 20 2004 009 521.1 bekannt, welches insbesondere für niedrigere Drücke ausgelegt ist.

Beim praktischen Einsatz dieser bekannten Ventile, welche sich im wesentlichen durch die Bauart des zweiten Schlauchanschlussgehäuses entsprechend der Druckbereiche, in denen sie zum Einsatz gelangen sollen, unterscheiden, haben sich gewisse praktische Probleme ergeben. Derartige Rückschlagventile werden in Krankenhäusern, insbesondere bei Infusionsbestecken, eingesetzt, wobei in Abhängigkeit von der speziellen Konfiguration des Infusionsbestecks diese Rückschlagventile manchmal sehr hohen Drücken in der Rückstromrichtung ausgesetzt werden können, welche Werte bis zu 325 psi (22,4 bar) erreichen können. Bei derartig hohen Drücken in der Rückstromrichtung wird die Membranscheibe aus dem flexiblen Werkstoff, bei welchem es sich meistens um Silikon handelt, innerhalb des Rückschlagventils entgegen der Einströmrichtung stark ausgelenkt.

Bei den beiden bekannten Rückschlagventilen der oben angegebenen Art ist der Einlass in dem ersten Schlauchanschlussgehäuse als einfache offene Bohrung ausgebildet, welche von dem Ventilsitz umgeben wird. Falls hier dann die oben erwähnten hohen Drücke in Rückwärtsrichtung auftreten, kann die Membranscheibe rückwärts nach unten in die offene Bohrung ausgelenkt werden und hierbei über ihre elastischen Grenzen gedehnt werden. Dieses kann wiederum zum Verlust der Rückschlagventilfunktion führen.

Ausgehend von einem Rückschlagventil der oben genannten Art liegt der Erfindung daher die Aufgabe zugrunde, das bekannte Ventil dahingehend zu verbessern, dass es gegenüber hohen Drücken entgegen der Einströmrichtung unempfindlich wird und insbesondere eine Beschädigung der Membranscheibe nicht mehr auftreten kann.

### Darstellung der Erfindung

Diese Aufgabe wird erfindungsgemäß dadurch gelöst, dass auf der Einlassseite in dem Einlassraum eine der Membranscheibe zugewandte mediendurchlässige Formation angeordnet ist, welche bei hohen Rückschlagdrücken die Membranscheibe gegen eine Überdehnung abstützt. Es ist offensichtlich, dass hierdurch die oben erwähnten Probleme vollständig vermieden werden.

Bei einer bevorzugten Ausführungsform nach der Erfindung ist diese Formation kreuzförmig in dem Einlassraum des Ventils ausgebildet.

Im Einzelnen kann die Erfindung dadurch weitergebildet werden, dass zwischen den die kreuzförmige Formation bildenden Streben kreissegmentförmige Einlassöffnungen ausgebildet sind.

Bei einer bevorzugten Ausführungsform nach der Erfindung ist die der Membranscheibe zugewandte Seite der kreuzförmigen Formation als ebene Stützfläche ausgebildet.

Im Einzelnen ist es von besonderem Vorteil, dass die gegen die Einströmrichtung gewandte Unterseite der Formation hydrodynamisch ausgebildet ist. Hierdurch wird Strömungsverluste und mögliche Verwirbelungen der hier durchströmenden medizinischen Flüssigkeiten vermieden.

Zu diesem Zweck sind bevorzugt auf der Unterseite der Formation aufeinander zulaufende Schrägflächen vorgesehen.

Bei einer besonders bevorzugten Ausführungsform nach der Erfindung ist vorgesehen, dass die Außenenden der die kreuzförmige Formation bildenden Streben in eine ringförmige Stützfläche übergehen, deren ebene Oberseite die der Membranscheibe zugewandte Stützfläche der Formation ergänzt. Hierdurch wird eine zusätzliche Sicherheit gegenüber Dehnung der Membran erreicht.

Im Einzelnen ist es besonders vorteilhaft, dass die Oberkante der Formation und der ringförmigen Stützfläche abgerundet oder mit einer Fase ausgebildet ist. Hierdurch wird eine besonders schonende Abstützung der Membranscheibe erreicht, so dass diese, selbst wenn sie unter hohem Druck in die Einlassöffnungen hineingedrückt wird, keinesfalls beschädigt werden kann.

Um den Strömungswiderstand des erfindungsgemäßen Rückschlagsventils in Durchlassrichtung weiter zu vermindern, ist es besonders bevorzugt, wenn die Formation radial in den Einlassraum ragende Arme umfasst.

Besonders bevorzugt ist es zu diesem Zweck, wenn sich die Arme im Zentrum des Einlassraumes nicht treffen, so dass dort ein Durchlass geringen Durchmessers offen bleibt.

Um trotzdem eine ausreichende Abstützkraft zu erreichen, ist es bevorzugt, wenn die Arme zur Mitte des Einlassraumes hin v-förmig zulaufend gestaltet sind.

Dabei ist es besonders bevorzugt, wenn die Arme so ausgebildet sind, dass sie zwischen sich nahezu rechteckige Schlitze mit parallelen Seitenkanten bilden.

Eine besonders gute Kraftverteilung ergibt sich, wenn sechs gleich beabstandete Arme vorgesehen sind.

Eine weitere Verminderung des Durchflusswiderstandes ergibt sich, wenn die Arme an ihren Vorderkanten abgeschrägt sind, sodass sich der Abstand zwischen den Armen in Durchflussrichtung vermindert.

Besonders bevorzugt ist es ferner, dass die Formation einstückig mit dem ersten Schlauchanschlussgehäuse ausgebildet ist.

### Kurze Beschreibung der Zeichnungen

Im Folgenden wird die Erfindung an Hand von in den Zeichnungen beispielhaft veranschaulichten Ausführungsformen näher erläutert. Es zeigt:
**FIGUR 1** eine seitliche Schnittansicht des erfindungsgemäßen Rückschlagventils im Ruhezustand;
**FIGUR 2** eine Figur 1 entsprechende Ansicht des Rückschlagventils während des Einströmens einer Flüssigkeit;
**FIGUR 3** eine Figur 1 und 2 entsprechende Ansicht des Rückschlagventils bei Auftreten eines erhöhten Drucks entgegen der Einströmrichtung;
**FIGUR 4** eine perspektivische Ansicht des ersten Schlauchanschlussgehäuses im vergrößerten Maßstab schräg von oben;
**FIGUR 5** eine perspektivische, teilweise geschnittene Ansicht von Figur 4;
**FIGUR 6** eine Draufsicht auf das erste Schlauchanschlussgehäuse gemäß Figur 4;
**FIGUR 7** eine perspektivische, teilweise geschnittene Ansicht von Figur 4 schräg von unten;
**FIGUR 8** zeigt eine seitliche Schnittansicht einer weiteren Ausführungsform des erfindungsgemäßen Rückschlagventils im Ruhezustand;
**FIGUR 9** eine Figur 8 entsprechende Ansicht der weiteren Ausführungsform des Rückschlagventils während des Einströmens einer Flüssigkeit;
**FIGUR 10** eine perspektivische Ansicht der weiteren Ausführungsform des ersten Schlauchanschlussgehäuses in vergrößertem Maßstab schräg von oben;
**FIGUR 11** eine perspektivische, teilweise geschnittene Ansicht von Figur 10;
**FIGUR 12** eine Draufsicht auf das erste Schlauchanschlussgehäuse gemäß Figur 10;
**FIGUR 13** eine perspektivische, teilweise geschnittene Ansicht von Figur 10 schräg von unten;
**FIGUR 14** das Detail der mediendurchlässigen Formation gemäß der weiteren Ausführungsform der vorliegenden Erfindung; und
**FIGUR 15** eine seitliche Schnittdarstellung des Details der Figur 14.

### Weg(e) zur Ausführung der Erfindung

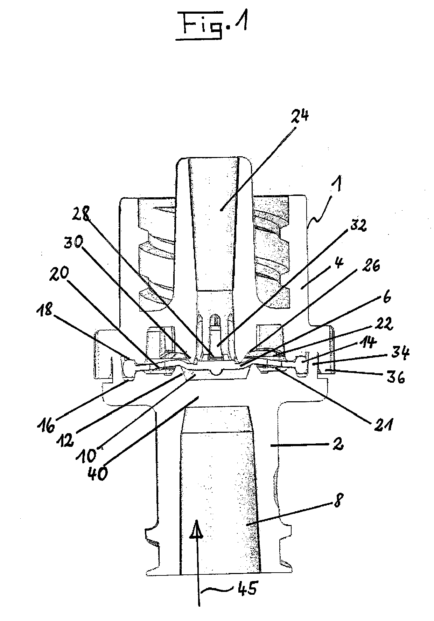

Das in den Figuren 1 bis 3 gezeigte Rückschlagventil 1 ist insbesondere für medizinische Zwecke geeignet und deckt beispielsweise Druckunterschiede von hohen Drücken bis herunter zu 0,002 bar ab. Das Rückschlagventil 1 besteht aus einem ersten Schlauchanschlussgehäuse 2 und einem zweiten Schlauchanschlussgehäuse 4, welche beispielsweise aus Kunststoff durch Spritzgießen hergestellt sind, sowie einer zwischen den beiden Schlauchanschlussgehäusen 2 und 4 angeordneten Membranscheibe 6, welche aus einem flexiblen Kunststoff, beispielsweise Silikon, besteht.

Das erste Schlauchanschlussgehäuse 2 weist einen Eingangsdurchlass 8 auf, welcher in einem Einlassraum 10 mündet. Der Einlassraum 10 ist von einem ringförmigen Ventilsitz 12 umgeben, welcher gegen die Membranscheibe 6 vorgespannt ist.

Die Membranscheibe 6 ist in ihrem, innerhalb des Ventilsitzes 12 liegenden Bereich ohne Öffnungen durchgehend ausgebildet, so dass erhebliche Zugkräfte radial von innen nach außen und umgekehrt übertragen werden können. Die Membranscheibe 6 ist an ihrem äußersten Umfangsbereich mit einem Ringwulst 14 versehen, welcher beispielsweise an die Membranscheibe 6 angespritzt ist, falls diese ebenfalls durch Spritzgießen des Silikons hergestellt wurde. In dem ersten Schlauchanschlussgehäuse 2 ist nahe deren Außenrand in der Stirnfläche des Schlauchanschlussgehäuses 2 eine Ringnut 16 ausgebildet, welcher eine Ringnut 18 des zweiten Schlauchanschlussgehäuses 4 im zusammengebauten Zustand gegenüberliegt. Wird das erste Schlauchanschlussgehäuse 2 mit dem zweiten Schlauchanschlussgehäuse 4 während der Montage des Rückschlagventils 1 beispielsweise durch Kleben oder Ultraschallschweißen verbunden, so wird der Ringwulst 14 in den einander gegenüberliegenden Ringnuten 16 und 18 der beiden Schlauchanschlussgehäuse 2 und 4 aufgenommen und dabei gleichzeitig die Membranscheibe 6 gegen den Ventilsitz 12 vorgespannt.

Wie in den Figuren 1 bis 3 ferner gezeigt, ist die Membranscheibe 6 radial außerhalb des Ventilsitzes 12 mit auf einem Radius angeordneten Öffnungen 20 versehen, die einen radial außerhalb des Ventilsitzes 12 liegenden Ringraum 21 im ersten Schlauchanschlussgehäuse 2 mit einem Auslassraum 22 des zweiten Schlauchanschlussgehäuses 4 verbinden, welcher seinerseits mit dem Ausgangsdurchlass 24 des zweiten Schlauchanschlussgehäuses 4 in Verbindung steht.

Der Auslassraum 22 ist nach oben durch die Wandung 26 des zweiten Schlauchanschlussgehäuses 4, die der Membranscheibe 6 gegenüberliegt, nach oben begrenzt. An der Wandung 26 sind allgemein mit 28 bezeichnete vorstehende mediendurchlässige Formationen vorgesehen, welche die Membranscheibe 6 gegen den Öffnungsdruck abstützen und gleichzeitig die Membranscheibe 6 in Richtung des Einlassraums 10 vorspannen, wobei die Formation 28 innerhalb der durch den Ventilsitz 12 gegebenen Grenze liegt.

Die Formation 28 besteht aus einer Anzahl von Vorsprüngen 30, welche kronenförmig die von dem Auslassraum 22 ausgehende Eingangsöffnung 32 des Ausgangsdurchlasses 24 umgeben.

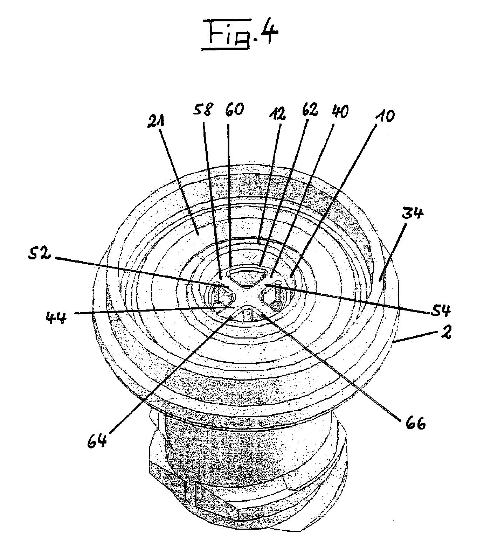

Zur Montage sind die beiden Schlauchanschlussgehäuse 2 und 4 mittels eines innen liegenden Ringvorsprungs 34 am ersten Schlauchanschlussgehäuse 2 und einem außen liegenden Ringvorsprung 36 am zweiten Schlauchanschlussgehäuse 4 ineinander greifend verbindbar. Nach Herstellung dieser Verbindung kann die endgültige Montage beispielsweise durch Ultraschallschweißen erfolgen. In den Figuren 1 bis 3 ist auf der Einlassseite in den Einlassraum 10 eine der Membranscheibe 6 zugewandte mediendurchlässige Formation 40 dargestellt, welche bei hohen Rückschlagdrücken die Membranscheibe 6 gegen eine Überdehnung abstützt. Diese mediendurchlässige Formation 40 wird weiter unten unter Bezugnahme auf die Figuren 4 bis 7 näher erläutert.

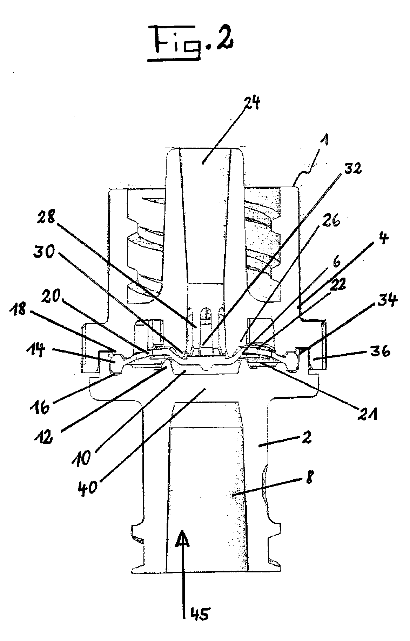

Bei der Darstellung in Figur 1 ist das Rückschlagventil 1 in seinem geschlossenen Ruhezustand veranschaulicht. Bei der Darstellung von Figur 2 strömt in Richtung des Pfeils 45 eine beispielsweise medizinische Flüssigkeit durch das Rückschlagventil 1, so dass die Membranscheibe 6 von dem Ventilsitz 12 abgehoben ist und die Flüssigkeit durch die Öffnungen 20 in der Membranscheibe 6 und die kronenförmige Formation 28 zum Ausgangsdurchlass strömen kann.

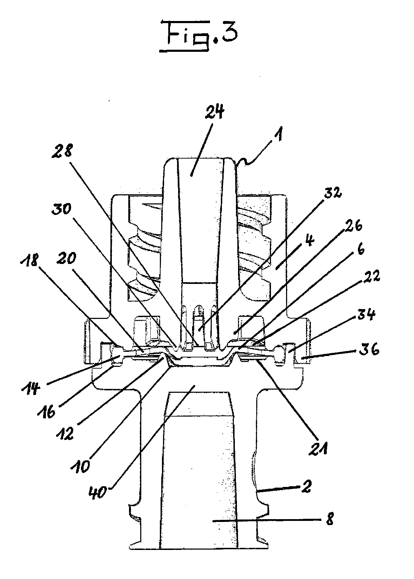

In Figur 3 ist der Zustand dargestellt, wenn in dem Rückschlagventil 1 ein starker Rückschlagdruck vom Ausgangsdurchlass 24 in Richtung des Eingangsdurchlasses 8 auftritt. In diesem Zustand wird, wie in Figur 3 dargestellt, die Membranscheibe 6 nach unten in den Einlassraum 10 hinein verformt und in dem Einlassraum 10 zur Vermeidung einer Überdehnung durch die Formation 40 abgestützt.

In den Figuren 4 bis 7, auf welche im Folgenden Bezug genommen wird, ist in näheren Einzelheiten das erste Schlauchanschlussgehäuse 2 mit der darin enthaltenen und einstückig ausgebildeten mediendurchlässigen Formation dargestellt.

Wie gezeigt, ist bei der dargestellten bevorzugten Ausführungsform die Formation 40 in der Mündung des Eingangsdurchlasses 8 in den Einlassraum 10 kreuzförmig ausgebildet. Die die kreuzförmige Formation 40 bildenden Streben 52 und 54 lassen kreissegmentförmige Einlassöffnungen 56 frei, durch welche die medizinische Flüssigkeit vom Eingangsdurchlass 8 in den Einlassraum 10 strömen kann.

Die der Membranscheibe 6 zugewandte Seite 42, das heißt in der Darstellung von Figur 4 die Oberseite der kreuzförmigen Formation 40, ist als ebene Stützfläche 44 für die Unterseite der Membranscheibe 6 ausgebildet.

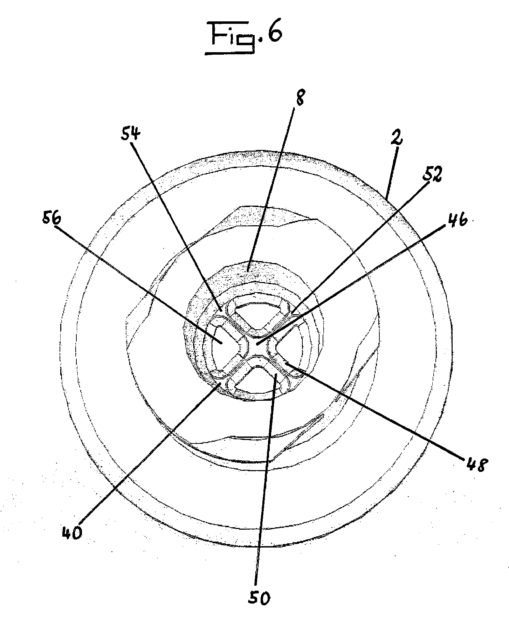

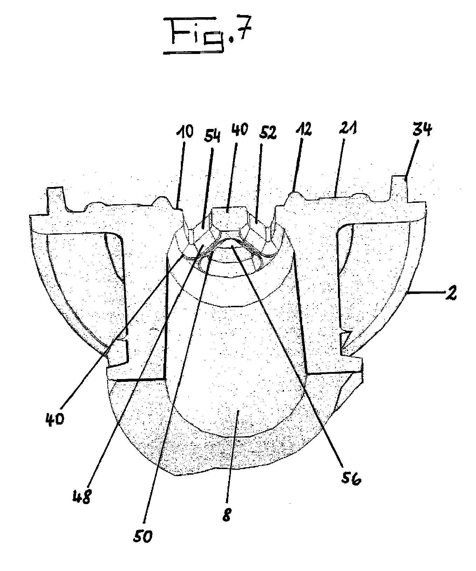

Wie ferner aus Figur 6 und 7 ersichtlich, ist die gegen die Einströmrichtung 45 zugewandte Unterseite 46 der Formation 40, das heißt der Streben 52 und 54, hydrodynamisch ausgebildet, wobei dies bei der veranschaulichten Ausführungsform dadurch geschehen ist, dass auf der Unterseite 46 der Formation 40 aufeinander zulaufende Schrägflächen 48 und 50 vorgesehen sind.

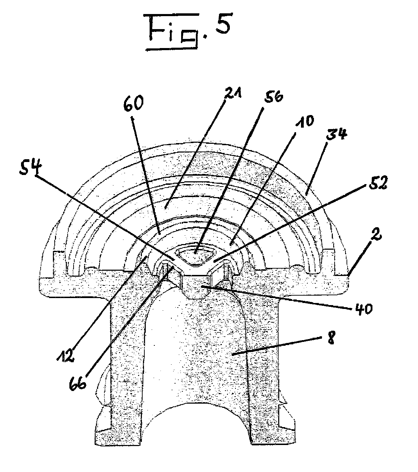

Um die Abstützung der Membranscheibe 6 bei zu hohen Rückschlagdrücken nochmals zu verbessern, gehen, wie aus Figur 4 und 5 ersichtlich, die Außenenden 58 der die kreuzförmige Formation 40 bildenden Streben 52 und 54 in eine ringförmige Stützfläche 60 über, deren ebene Oberseite 62 die der Membranscheibe 6 zugewandte Stützfläche 44 der Formation 40 ergänzt.

Aus den Figuren 4 und 5 ist ferner ersichtlich, dass bevorzugt die Oberkante 64 der Formation 40 und der ringförmigen Stützfläche 60 abgerundet oder mit einer Fase 66 ausgebildet ist, um zu vermeiden, dass die Membranscheibe 6 beschädigt wird, falls der Rückschlagdruck so groß ist, dass Bereiche der Membranscheibe 6 in die Einlassöffnungen 56 gedrückt werden.

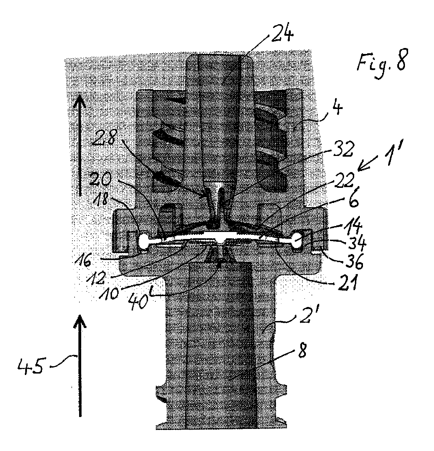

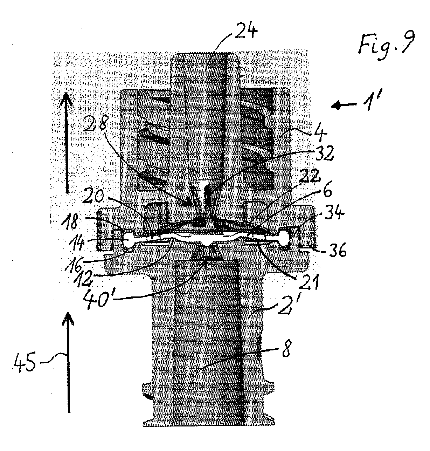

Die Figuren 8 und 9 zeigen eine weitere Ausführungsform für ein erfindungsgemäßes Rückschlagventil 1'. Dieses weist einen besonders geringen Strömungswiderstand in Durchlassrichtung auf. Das erfindungsgemäße Rückschlagventil 1' besteht aus einem ersten Schlauchanschlussgehäuse 2' und einem zweiten Schlauchanschlussgehäuse 4, welche beispielsweise aus Kunststoff durch Spritzgießen hergestellt sein können, sowie einer zwischen den beiden Schlauchanschlussgehäusen 2' und 4 angeordneten Membranscheibe 6, welche aus einem flexiblen Kunststoff, beispielsweise Silikon, besteht.

Hier und im folgenden werden für gleiche Gegenstände die gleichen Bezugszeichen verwendet, um die Verständlichkeit der Beschreibung und die Vergleichbarkeit der beiden Ausführungsformen der Erfindung zu verbessern. Die Bezugszeichen für abgewandelte Bestandteile der weiteren Ausführungsform sind mit einem Apostroph' versehen.

Auch bei dieser zweiten Ausführungsform weist das erste, in den Zeichnungen untere Schlauchanschlussgehäuse 2' einen Eingangsdurchlass 8 auf, welcher in einen Einlassraum 10 mündet. Der Einlassraum 10 ist von einem ringförmigen Ventilsitz 12 umgeben, gegen den die Membranscheibe 6 vorgespannt ist.

Die Membranscheibe 6 ist in ihrem innerhalb des Ventilsitzes 12 liegenden Bereich ohne Öffnungen durchgehend ausgebildet, so dass erhebliche Zugkräfte radial von innen nach außen und umgekehrt übertragen werden können. Die Membranscheibe 6 ist an ihrem äußersten Umfangsbereich mit einem Ringwulst 14 versehen, welcher beispielsweise an die Membranscheibe 6 angespritzt ist, falls diese ebenfalls durch Spritzgießen des Silikons hergestellt wurde. In dem ersten Schlauchanschlussgehäuse 2' ist nahe dessen Außenrand in der Stirnfläche des Schlauchanschlussgehäuses 2 eine Ringnut 16 ausgebildet, welcher eine Ringnut 18 des zweiten Schlauchanschlussgehäuses 4 in zusammengebautem Zustand gegenüberliegt. Wird das erste Schlauchanschlussgehäuse 2' mit dem zweiten Schlauchanschlussgehäuse 4 während der Montage des Rückschlagventils 1' beispielsweise durch Kleben oder Ultraschallschweißen verbunden, so wird der Ringwulst 14 in den einander gegenüberliegenden Ringnuten 16 und 18 der beiden Schlauchanschlussgehäuse 2' und 4 aufgenommen und dabei gleichzeitig die Membranscheibe 6 gegen den Ventilsitz 12 vorgespannt.

Wie in den Figuren 8 und 9 dargestellt, ist die Membranscheibe 6 radial außerhalb des Ventilsitzes 12 mit auf einem Radius angeordneten Öffnungen 20 versehen, die einen radial außerhalb des Ventilsitzes 12 liegenden Ringraum 21 im ersten Schlauchanschlussgehäuse 2' mit einem Auslassraum 22 des zweiten Schlauchanschlussgehäuses 4 verbinden, welcher seinerseits mit dem Ausgangsdurchlass 24 des zweiten Schlauchanschlussgehäuses 4 in Verbindung steht.

Der Auslassraum 22 ist über einen kronenförmige Formation 28, die eine Mehrzahl von in Durchflussrichtung verlaufenden Schlitzen 32 aufweist, mit dem Ausgangsdurchlass 24 verbunden. Diese kronenförmige Formation stützt die Membranscheibe 6 im Bedarfsfall gegen den Öffnungsdruck ab.

Zur Montage sind die beiden Schlauchanschlussgehäuse 2' und 4 mittels eines innenliegenden Ringvorsprungs 34 am ersten Schlauchanschlussgehäuse 2' und einem außenliegenden Ringvorsprung 36 am zweiten Schlauchanschlussgehäuse 4 ineinandergreifend verbindbar. Nach Herstellung dieser Verbindung kann die endgültige Montage beispielsweise durch

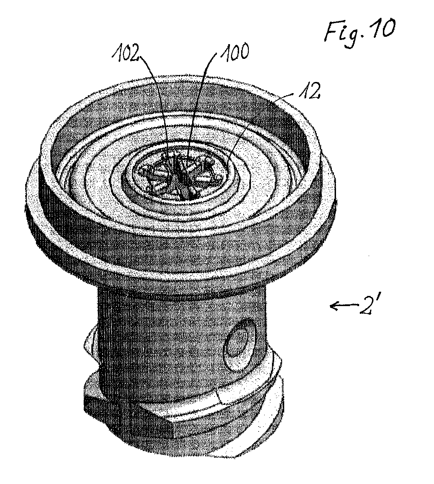

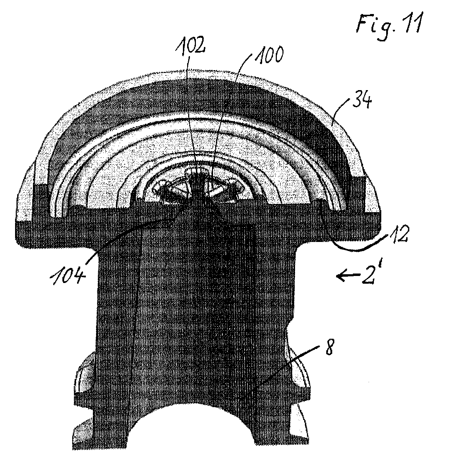

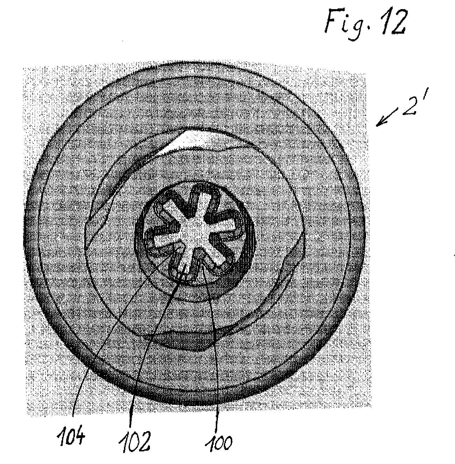

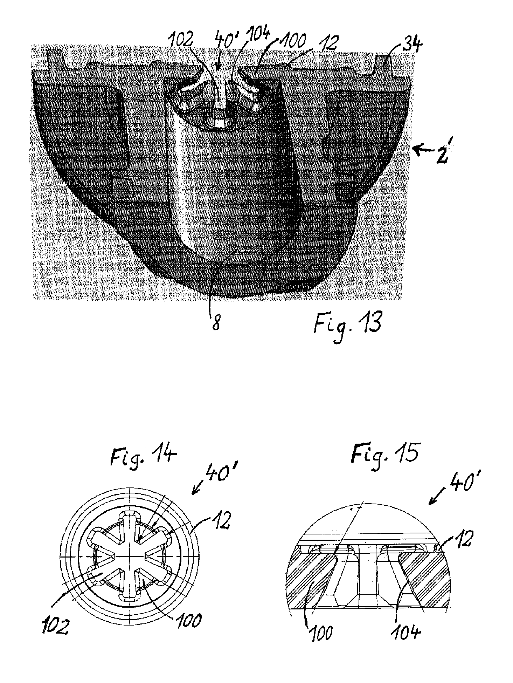

Ultraschallschweißen erfolgen. In den Figuren 8 und 9 ist auf der Einlassseite in den Einlassraum 10 eine der Membranscheibe 6 zugewandte mediendurchlässige Formation 40' dargestellt, welche bei hohen Rückschlagdrücken die Membranscheibe 6 gegen eine Überdehnung abstützt. Diese mediendurchlässige Formation 40' ist bei der hier dargestellten Ausführungsform anders ausgebildet als bei der Ausführungsform der Figuren 1 bis 7. Sie wird weiter unten unter Bezugnahme auf die Figuren 10 bis 15 näher erläutert.

In Fig. 8 ist das Rückschlagventil 1' in seinem geschlossenen Ruhezustand dargestellt. In Fig. 9 strömt eine Flüssigkeit in Richtung des Pfeils 45 durch das Rückschlagventil 1', so dass die Membranscheibe 6 von dem Ventilsitz 12 abgehoben ist, und die Flüssigkeit durch die Öffnungen 20 in der Membranscheibe 6 und die kronenförmige Formation 28 zum Ausgangsdurchlass strömen kann.

In den Figuren 10 bis 15, auf die die Beschreibung im folgenden Bezug nimmt, ist das erste Schlauchanschlussgehäuse 2' mit der in dieser Ausführungsform deutlich anders ausgebildeten mediendurchlässigen Formation 40' dargestellt.

In dieser weiteren Ausführungsform der Erfindung umfasst die Formation 40' einstückig mit dem Schlauchanschlussgehäuse 2' ausgebildete Arme 100, die sich vom Ventilsitz 12 aus radial nach innen erstrecken. Diese Arme 100 erstrecken sich dabei nicht ganz bis zur Mitte des Einlassraums 10, sondern die radial inneren Enden der Arme 100 halten einen gewissen Abstand voneinander ein, so dass zwischen diesen Armen 100 ein runder Durchlass mit geringerem Durchmesser offen bleibt. Die Arme 100 selbst weisen eine nach innen zulaufende V-förmige Struktur auf, die so gestaltet ist, dass zwischen den Armen Schlitze 102 vorhanden sind, die eine im wesentliche rechteckige Form mit nahezu parallelen Seitenkanten aufweisen. Alternativ können diese Seitenkanten auch radial verlaufen. Bei der vorliegenden Ausführungsform sind sechs Arme 100 vorgesehen.

Die Vorderkanten der Arme 100 sind dergestalt abgeschrägt, dass sich der Durchlassraum zwischen den Armen in Durchflussrichtung zunehmend verengt. Von dort gehen die Schrägen 104 abgerundet in die Oberfläche der Arme 100 über.

Bei dieser weiteren Ausführungsform der Erfindung wird durch die spezielle Ausgestaltung der Arme 100 sowie die zusätzliche Abschrägung 104 ein wesentlich geringerer Strömungswiderstand in Durchflussrichtung erzielt als bei der Ausführungsform gemäß Fig. 1 bis 8.

### BEZUGSZEICHENLISTE

- 1, 1' =: Rückschlagventil
- 2, 2' =: erstes Schlauchanschlussgehäuse
- 4 =: zweites Schlauchanschlussgehäuse
- 6 =: Membranscheibe
- 8 =: Eingangsdurchlass
- 10 =: Einlassraum
- 12 =: Ventilsitz
- 14 =: Ringwulst
- 16 =: Ringnut
- 18 =: Ringnut
- 20 =: Öffnungen
- 21 =: Ringraum
- 22 =: Auslassraum
- 24 =: Ausgangsdurchlass
- 26 =: Wandung
- 28 =: kronenförmige Formation
- 30 =: Vorsprünge
- 32 =: Eingangsöffnung
- 34 =: Ringvorsprung
- 36 =: Ringvorsprung
- 40, 40' =: Formation
- 42 =: Seite von 40
- 44 =: Stützfläche
- 45 =: Eintrittsöffnung
- 46 =: Unterseite von 40
- 48 =: Schrägfläche
- 50 =: Schrägfläche
- 52 =: Strebe von 28
- 54 =: Strebe von 28
- 56 =: Einlassöffnung
- 58 =: Außenende von 52, 54
- 60 =: ringförmige Stützfläche
- 62 =: Oberseite von 60
- 64 =: Oberkante von 40
- 66 =: Fase
- 100 =: Arme
- 102 =: Schlitze
- 104 =: abgeschrägte Vorderkanten

## Patentansprüche

1. Rückschlagventil, insbesondere für medizinische Anwendungen, mit einem ersten Schlauchanschlussgehäuse (2) und einem zweiten Schlauchanschlussgehäuse (4) und einer zwischen den beiden Schlauchanschlussgehäusen angeordneten Membranscheibe (6) aus flexiblem Werkstoff, welche bei Überdruck in einem Eingangsdurchlass im ersten Schlauchanschlussgehäuse von einem ringförmigen, einen mit dem Eingangsdurchlass verbundenen Einlassraum (10) umgebenden Ventilsitz (12) abhebbar ist und die bei Überdruck in einem Ausgangsdurchlass (24) sicher und in Minimalzeiten auf dem Ventilsitz andrückbar ist, wobei die Membranscheibe an ihrem äußeren Umfangsbereich mit einem Ringwulst (14) versehen ist, welcher in einander gegenüberliegenden Ringnuten (16, 18) der Schlauchanschlussgehäuse aufgenommen ist, wobei die Membranscheibe (6) radial außerhalb des Ventilsitzes (12) mit Öffnungen (20) versehen ist, die mit einem Auslassraum (22) verbunden sind, **dadurch gekennzeichnet, dass** auf der Einlassseite in dem Einlassraum (10) eine der Membranscheibe (6) zugewandte mediendurchlässige Formation (40; 40') angeordnet ist, welche bei hohen Rückschlagdrücken die Membranscheibe (6) gegen eine Überdehnung abstützt.

2. Rückschlagventil nach Anspruch 1, **dadurch gekennzeichnet, dass** die Formation (40) kreuzförmig mittels Streben (52, 54) in dem Einlassraum (10) ausgebildet ist.

3. Rückschlagventil nach Anspruch 2, **dadurch gekennzeichnet, dass** zwischen den die kreuzförmige Formation (40) bildenden Streben (52, 54) kreissegmentförmige Einlassöffnungen (56) ausgebildet sind.

4. Rückschlagventil nach Anspruch 2 oder 3, **dadurch gekennzeichnet, dass** die der Membranscheibe (6) zugewandte Seite (42) der kreuzförmigen Formation (40) als ebene Stützfläche (44) ausgebildet ist.

5. Rückschlagventil nach einem der vorstehenden Ansprüche, **dadurch gekennzeichnet, dass** die gegen die Einströmrichtung (45) zugewandte Unterseite (46) der Formation (40) hydrodynamisch ausgebildet ist.

6. Rückschlagventil nach Anspruch 5, **dadurch gekennzeichnet, dass** auf der Unterseite (46) der Formation (40) aufeinander zulaufende Schrägflächen (48, 50) vorgesehen sind.

7. Rückschlagventil nach einem der Ansprüche 2 bis 6, **dadurch gekennzeichnet, dass** die Außenenden (58) der die kreuzförmige Formation (40) bildenden Streben (52, 54) in eine ringförmige Stützfläche (60) übergehen, deren ebene Oberseite (62) die der Membranscheibe (6) zugewandte Stützfläche (44) der Formation (40) ergänzt.

8. Rückschlagventil nach einem der vorstehenden Ansprüche, **dadurch gekennzeichnet, dass** die Oberkante (64) der Formation (40) und der ringförmigen Stützfläche (60) abgerundet oder mit einer Fase (66) ausgebildet ist.

9. Rückschlagventil nach Anspruch 1, **dadurch gekennzeichnet, dass** die Formation (40') radial in den Einlassraum (10) ragende Arme (100) umfasst.

10. Rückschlagventil nach Anspruch 9, **dadurch gekennzeichnet, dass** die Arme (100) sich im Zentrum des Einlassraumes (10) nicht treffen, so dass dort ein Durchlass geringeren Durchmessers offen bleibt.

11. Rückschlagventil nach Anspruch 9 oder 10, **dadurch gekennzeichnet, dass** die Arme (100) zur Mitte des Einlassraumes (10) hin V-förmig zulaufend gestaltet sind.

12. Rückschlagventil nach Anspruch 11, **dadurch gekennzeichnet, dass** die Arme (100) so ausgebildet sind, dass sie zwischen sich nahezu rechteckige Schlitze (102) mit nahezu parallelen Seitenkanten bilden.

13. Rückschlagventil nach einem der Ansprüche 9 bis 12, **dadurch gekennzeichnet, dass** sechs gleich beabstandete Arme (100) vorgesehen sind.

14. Rückschlagventil nach einem der Ansprüche 10 bis 13, **dadurch gekennzeichnet, dass** die Arme (100) an ihren Vorderkanten (104) abgeschrägt sind, so dass sich der Abstand zwischen den Armen (100) in Durchflussrichtung vermindert.

15. Rückschlagventil nach einem der vorstehenden Ansprüche, **dadurch gekennzeichnet, dass** die Formation (40) einstückig mit dem ersten Schlauchanschlussgehäuse (2) ausgebildet ist.

## Claims

1. Check valve, in particular for medical applications, with a first tube connection housing (2) and a second tube connection housing (4) and, arranged between the two tube connection housings, a diaphragm disc (6) which is made of flexible material and which, when there is an overpressure in an inlet channel in the first tube connection housing, can be lifted from an annular valve seat (12) surrounding an inlet chamber (10) connected to the inlet channel, and which, when there is an overpressure in an outlet channel (24), can be pressed reliably and within minimal times onto the valve seat, wherein the diaphragm disc, at its outer peripheral area, is provided with an annular bead (14) which is received in mutually opposite annular grooves (16, 18) of the tube connection housings, wherein the diaphragm disc (6) is provided, radially outside the valve seat (12), with openings (20) connected to an outlet chamber (22), **characterized in that**, on the inlet side in the inlet chamber (10), a media-permeable formation (40; 40') is arranged which faces towards the diaphragm disc (6) and which, at high return pressures, supports the diaphragm disc (6) against overextension.

2. Check valve according to Claim 1, **characterized in that** the formation (40) is configured in a cross shape by means of struts (52, 54) in the inlet chamber (10).

3. Check valve according to Claim 2, **characterized in that** inlet openings (56) shaped as segments of a circle are formed between the struts (52, 54) forming the cross-shaped formation (40).

4. Check valve according to Claim 2 or 3, **characterized in that** the side (42) of the cross-shaped formation (40) facing towards the diaphragm disc (6) is designed as a plane support surface (44).

5. Check valve according to one of the preceding claims, **characterized in that** the underside (46) of the formation (40), facing against the inflow direction (45), is configured to be hydrodynamic.

6. Check valve according to Claim 5, **characterized in that** bevelled surfaces (48, 50) running towards each other are provided on the underside (46) of the formation (40).

7. Check valve according to one of Claims 2 to 6, **characterized in that** the outer ends (58) of the struts (52, 54) forming the cross-shaped formation (40) merge into an annular support surface (60), of which the plane upper face (62) supplements the support surface (44) of the formation (40) facing towards the diaphragm disc (6).

8. Check valve according to one of the preceding claims, **characterized in that** the upper edge (64) of the formation (40) and of the annular support surface (60) is rounded or provided with a chamfer (66) .

9. Check valve according to Claim 1, **characterized in that** the formation (40') comprises arms (100) protruding radially into the inlet chamber (10).

10. Check valve according to Claim 9, **characterized in that** the arms (100) do not meet at the centre of the inlet chamber (10), such that a channel of smaller diameter remains open there.

11. Check valve according to Claim 9 or 10, **characterized in that** the arms (100) are configured narrowing in a V shape towards the centre of the inlet chamber (10).

12. Check valve according to Claim 11, **characterized in that** the arms (100) are configured such that between them they form almost rectangular slits (102) with almost parallel side edges.

13. Check valve according to one of Claims 9 to 12, **characterized in that** six arms (100) at equal distances from one another are provided.

14. Check valve according to one of Claims 10 to 13, **characterized in that** the arms (100) are bevelled at their front edges (104), such that the distance between the arms (100) decreases in the direction of flow.

15. Check valve according to one of the preceding claims, **characterized in that** the formation (40) is configured in one piece with the first tube connection housing (2).

## Revendications

1. Clapet anti-retour, en particulier pour applications médicales, comprenant un premier boîtier de raccordement de tuyaux (2) et un deuxième boîtier de raccordement de tuyaux (4) et un disque de membrane (6) disposé entre les deux boîtiers de raccordement de tuyaux, constitué de matériau flexible, qui peut être soulevé en cas de surpression dans un passage d'entrée dans le premier boîtier de raccordement de tuyaux depuis un siège de clapet annulaire (12) entourant un espace d'entrée (10) connecté au passage d'entrée et qui peut être pressé sur le siège de clapet en cas de surpression dans un passage de sortie (24) de manière sûre et en des temps minimaux, le disque de membrane étant muni au niveau de sa région périphérique extérieure d'un bourrelet annulaire (14) qui est reçu dans des rainures annulaires mutuellement opposées (16, 18) des boîtiers de raccordement de tuyaux, le disque de membrane (6) étant pourvu d'ouvertures (20) radialement à l'extérieur du siège de clapet (12), lesquelles ouvertures sont connectées à un espace de sortie (22), **caractérisé en ce que** du côté de l'entrée dans l'espace d'entrée (10) est disposée une formation (40 ; 40') perméable à des milieux et tournée vers le disque de membrane (6), laquelle supporte le disque de membrane (6) contre une surextension en cas de pressions de rappel élevées.

2. Clapet anti-retour selon la revendication 1, **caractérisé en ce que** la formation (40) est réalisée sous forme cruciforme au moyen d'entretoises (52, 54) dans l'espace d'entrée (10).

3. Clapet anti-retour selon la revendication 2, **caractérisé en ce qu'**entre les entretoises (52, 54) formant la formation cruciforme (40) sont réalisées des ouvertures d'entrée (56) en forme de segment de cercle.

4. Clapet anti-retour selon la revendication 2 ou 3, **caractérisé en ce que** le côté (42) de la formation cruciforme (40) tourné vers le disque de membrane (6) est réalisé sous forme de surface de support plane (44).

5. Clapet anti-retour selon l'une quelconque des revendications précédentes, **caractérisé en ce que** le côté inférieur (46) de la formation (40) tourné à l'opposé du sens de l'afflux (45) est réalisé sous forme hydrodynamique.

6. Clapet anti-retour selon la revendication 5, **caractérisé en ce que** sur le côté inférieur (46) de la formation (40) sont prévues des surfaces obliques (48, 50) convergeant l'une vers l'autre.

7. Clapet anti-retour selon l'une quelconque des revendications 2 à 6, **caractérisé en ce que** les extrémités extérieures (58) des entretoises (52, 54) formant la formation cruciforme (40) se prolongent par une surface de support annulaire (60) dont le côté supérieur plan (62) complète la surface de support (44) de la formation (40) tournée vers le disque de membrane (6).

8. Clapet anti-retour selon l'une quelconque des revendications précédentes, **caractérisé en ce que** le bord supérieur (64) de la formation (40) et de la surface de support annulaire (60) est réalisé sous forme arrondie ou avec un biseau (66).

9. Clapet anti-retour selon la revendication 1, **caractérisé en ce que** la formation (40') comprend des bras (100) faisant saillie radialement dans l'espace d'entrée (10).

10. Clapet anti-retour selon la revendication 9, **caractérisé en ce que** les bras (100) ne se rejoignent pas au centre de l'espace d'entrée (10), de sorte qu'il subsiste à cet endroit un passage ouvert de plus faible diamètre.

11. Clapet anti-retour selon la revendication 9 ou 10, **caractérisé en ce que** les bras (100) sont configurés de manière à converger en forme de V vers le centre de l'espace d'entrée (10).

12. Clapet anti-retour selon la revendication 11, **caractérisé en ce que** les bras (100) sont réalisés de telle sorte qu'ils forment entre eux des fentes pratiquement rectangulaires (102) avec des bords latéraux pratiquement parallèles.

13. Clapet anti-retour selon l'une quelconque des revendications 9 à 12, **caractérisé en ce que** six bras (100) espacés uniformément sont prévus.

14. Clapet anti-retour selon l'une quelconque des revendications 10 à 13, **caractérisé en ce que** les bras (100) sont biseautés au niveau de leurs bords avant (104), de telle sorte que la distance entre les bras (100) diminue dans la direction de l'écoulement.

15. Clapet anti-retour selon l'une quelconque des revendications précédentes, **caractérisé en ce que** la formation (40) est réalisée d'une seule pièce avec le premier boîtier de raccordement de tuyaux (2) .
